(19) Europäisches Patentamt
European Patent Office
Office européen des brevets

(11) Publication number: **0 101 760**
**B1**

(12) **EUROPEAN PATENT SPECIFICATION**

(45) Date of publication of patent specification: **12.02.86**

(51) Int. Cl.$^4$: **C 07 C 49/813**, C 07 C 49/84, C 07 C 45/63

(21) Application number: **82109476.0**

(22) Date of filing: **13.10.82**

(54) **Process for producing fluorobenzophenone derivatives.**

(43) Date of publication of application:
**07.03.84 Bulletin 84/10**

(45) Publication of the grant of the patent:
**12.02.86 Bulletin 86/07**

(84) Designated Contracting States:
**DE FR GB NL**

(56) References cited:
**EP-A-0 010 651**
**US-A-3 387 035**

**Patent Abstracts of Japan, vol. 7, no. 11, 18 January 1983**

**Chemical Abstracts, vol. 43, no. 21, 10 November 1949, Columbus, Ohio, USA, C.I. TEWKSBURY et al. "METAL FLUORIDES AS FLUORINATING AGENTS", COLUMN 8364g-h**

**J. Amer. Chem. Soc. 78, 6034-6037 (1956)**

(73) Proprietor: **MITSUI TOATSU CHEMICALS, INCORPORATED**
**2-5 Kasumigaseki 3-chome**
**Chiyoda-Ku Tokyo 100 (JP)**

(72) Inventor: **Yamaguchi, Akihiro**
**1-13-24, Zaimokuza**
**Kamakura-shi Kanagawa-ken (JP)**
Inventor: **Yamaguchi, Keizaburo Mitsui Toatsu Chemicals'**
**Apartment 533 600-1, Kamisakunobe Takatsu-ku**
**Kawasaki-shi Kanagawa-ken (JP)**
Inventor: **Sugimoto, Kenichi**
**105, Minezawa-cho Hodogaya-ku**
**Yokohama-shi Kanagawa-ken (JP)**
Inventor: **Tanabe, Yoshimitsu**
**2070, Iizima-cho Totsuka-ku**
**Yokohama-shi Kanagawa-ken (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

**EP 0 101 760 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the production of fluorobenzophenone derivatives and particularly, 4-fluorobenzophenone derivatives and 4,4'-difluorobenzophenone.

Fluorobenzophenone derivatives, for example 4,4'-difluorobenzophenone, are used as materials for producing an aromatic polyketone polymer and 4-fluorobenzophenone derivatives are useful as agricultural chemicals and drugs and intermediates for dyestuffs.

Generally fluoro organic compounds, such as fluorinated aliphatic hydrocarbons and derivatives thereof may be produced by an exchange reaction of the corresponding chloro-, bromo-, or iodo-aliphatic compounds with metal fluorides, such as potassium fluoride or calcium fluoride. According to EP—A—0010651 aliphatic fluorides are produced by reacting the corresponding chlorides or bromides with calcium chloride in the presence of a cage compound and an anhydrous polar basic organic solvent. The literature has, however, shown that the known halogen exchange reaction between metal fluorides and halogen substituted organic compounds, when applied in the case of aromatic compounds, can only be carried out successfully in very specific cases with regard to the metal fluoride and with regard to the substitution of the starting aromatic halogeno compound. According to Chemical Abstracts Vol. 43, No. 21, November 1949, column 8364 g-h, α,α,α-trichloro toluene may be converted to α,α,α-trifluoro toluene by a vapour phase reaction at 225°C in a copper reaction vessel in the presence of NaF, $ZnF_2$, $CdF_2$, $CoF_2$, $PbF_2$, $SbF_3$, $BiF_3$ and $CuF_2$, while the fluorides of Li, K, Ca, Mg, Al and Mn are not effective in the reaction. Another evidence that generally unactivated aryl halides do not undergo the exchange reaction between the organic halide and inorganic fluorides is the publication by Finger and Kruse in J. Am. Chem. Soc., *78,* p. 6034 to 6037 (1956). It is expressly stated that the activation by at least two nitro groups has been considered necessary for exchange between aryl halides and fluoride ion. According to the stated publication the reaction could be expanded to 4-nitrochlorobenzene when using a specific polar solvent for the reaction. From this prior art it had to be concluded that generally this exchange reaction may not be applied to aromatic halides, except specific cases. Obviously for that reason several different and rather complicated processes have been used for the production of fluorinated benzophenone derivatives.

For producing 4,4'-difluorobenzophenone there have been known, for example, (1) a process of reacting fluorobenzene with carbon tetrachloride in the presence of anhydrous aluminum chloride and then hydrolyzing [J. P. Picard et al, Can. J. Research, *28B,* 56 (1950); C. A., *44,* 4442a (1950); Funasaka et al, Yukigoseikagaku Kyokaishi, *17,* 334 (1959)], (2) a process of effecting a Friedel-Craft's reaction between p-fluorobenzoylchloride and fluorobenzene in the presence of anhydrous aluminum chloride [R. C. Iris et al, Rev. inst. Salubridad y enfermeded. trop., *8,* 63 (1947); C. A., *42,* 1921 a (1948)], (3) a process of oxidizing 2,2'-bis(4-fluorophenyl)-1,1-dichloroethylene with chromic acid [H. L. Bradlow et al, J. Am. Chem. Soc., 69, 662 (1947)] and (4) a process of treating 4,4'-diaminodiphenylmethane with sodium nitrite in anhydrous hydrogen fluoride or in a concentrated aqueous solution of hydrogen fluoride (Japanese Patent Application Kokai No. 54—132, 558).

Also, for producing 4-fluorobenzophenone derivatives there have been known, for example, (5) a process of effecting a Friedel-Crafts' reaction between a substituted benzoylchloride and fluorobenzene in the presence of anhydrous aluminum chloride [R. D. Dunlop et al, J. Am. Chem. Soc., *55,* 1665 (1933); K. C. Joshi et al, J. Indian Chem. Soc., *40,* 42 (1963); R. F. Pews et al, J. Am. Chem. Soc., *89,* 2392 (1967)] and (6) a process of reacting p-fluorobenzoylchloride with an aromatic hydrocarbon in the presence of anhydrous aluminum chloride [Z. Eckstein et al, Bull. acad. polon. sci., Ser. sci., chim., geol. et geograph., *7,* 803 (1959); Chem. Abstr. *54,* 21005 b (1960)].

However, in case of the processes (1) and (2) fluorobenzene is an expensive material and anhydrous aluminum chloride in a more than equimol relation to fluorobenzene is required. Also, the separation of the catalyst is too complicated to reuse it. Further, an apparatus made of anticorrosive material is required.

In case of the process (3) fluorobenzene is used as a starting material and a large amount of glacial acetic acid is necessary. Also, since an excess of anhydrous chromic acid is used, the expenses for treating the generated waste to become non-polluting are important.

In case of the process (4) the starting materials are of relatively low costs, while since a large excess of anhydrous and concentrated hydrogen fluoride is used, the recovery of hydrogen fluoride is necessary.

Further, for the production an apparatus made of specific materials such as a reaction and recovery vessel of anti-corrosive materials is required and also, the toxicity of hydrogen fluoride should be taken into consideration.

In case of the processes (5) and (6) fluorobenzene and p-fluorobenzoylchloride are industrially expensive and anhydrous aluminum chloride in more than the equimolar amount is necessary. The separation of the catalyst is too complicated to reuse it and also, the load for treating the generated waste fluid is heavy. Therefore, the application of these processes in a commercial scale is accomplished with various difficulties.

Regarding the described prior art it is therefore an object of this invention to provide a process for producing fluorobenzophenone derivatives conveniently in a commercial scale starting from the corresponding halogenobenzophenone derivatives.

In accordance with this invention there is provided a process for producing 4-fluorobenzophenone

derivatives which comprises effecting a halogen-fluorine exchange reaction between 4-halogenobenzophenone derivatives of Formula I,

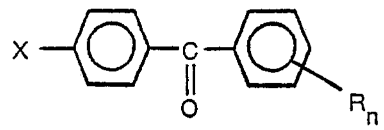

I

wherein X is chlorine, bromine or iodine atom, R is hydrogen, an alkyl group, an aralkyl group, a cycloalkyl group, an aryl group, an alkoxy group, an aryloxy group, an alkylthio group, a carboalkoxy group, an acetyl group, a trifluoromethyl group, a nitro group or a chlorine, bromine or iodine atom, n is an integer of 1—5 and when n is 2 or more, R is the same or different, and an alkali fluoride in an organic solvent at a temperature of 100°C to 350°C.

In a specific embodiment of the present invention the halogen-fluorine exchange is effected between a 4,4'-dihalogenobenzophenone of the formula

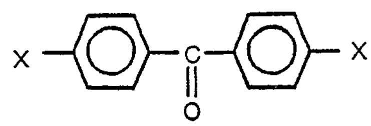

Ia

wherein X is chlorine, bromine or iodine, and an alkali fluoride. In the following the invention is described in more detail.

Acording to this invention, the corresponding 4-fluorobenzophenone derivatives and 4,4-difluorobenzophenone are produced with a high yield by heating the 4-halogenobenzophenone derivative of formula I or Ia respectively and an alkali fluoride in an organic solvent. After distilling off the solvent from the reaction product mixture, the end product is isolated easily by extracting with a solvent. Alternatively, the end product is isolated by distillation. Therefore the process of this invention is quite advantageous industrially.

The 4-halogenobenzophenone derivatives of Formula I which may be used in this invention are, for example, 4-halogenobenzophenone, 4-halogeno-3'-methylbenzophenone, 4-halogeno-4'-methyl-benzophenone, 4-halogeno-4'-ethylbenzophenone, 4-halogeno-4'-n-butylbenzophenone, 4-halogeno-2',4'-dimethylbenzophenone, 4-halogeno-2',5'-dimethylbenzophenone, 4-halogeno-4'-phenylbenzophenone, 4-halogeno-4'-methoxybenzophenone, 4-halogeno-4'-ethoxybenzophenone, 4-halogeno-4'-phenoxybenzophenone, 4-halogeno-4'-methylthiobenzophenone, 4-halogeno-3'-carbomethoxy-benzophenone, 4-halogeno-3'-acetylbenzophenone, 4-halogeno-3'-trifluoromethylbenzophenone, 4-halogeno-3'-nitrobenzophenone, 4-halogeno-4'-nitrobenzophenone, 2-chloro-4'-halogenobenzophenone, 2-bromo-4'-halogenobenzophenone, 3-chloro-4'-halogenobenzophenone, 4-halogeno-3',5'-dichlorobenzophenone. Useful 4,4'-dihalogenobenzophenones of the formula Ia are 4,4'-dichlorobenzophenone, 4,4'-dichlorobenzophenone, 4,4'-dibromobenzophenone and 4,4'-diiodobenzophenone.

These 4-halogenobenzophenone derivatives of Formula I may be prepared by effecting a Friedel-Craft's reaction between p-chloro, p-bromo- or p-iodo-benzoyl chloride and an aromatic hydrocarbon which are both readily available in a commercial scale or between a substituted benzoylchloride and chlorobenzene, bromobenzene or iodobenzene in the presence of an anhydrous ferric chloride catalyst.

The alkali fluorides which may be used in this invention are, for example, cesium fluoride, rubidium fluoride, potassium fluoride, sodium fluoride and lithium fluoride. Taking into consideration the yield of end product cesium fluoride, rubidium fluoride and potassium fluoride are preferred. These may be used in mixture of two or more. The amount of the alkali fluoride for attaining the halogen-fluoride exchange reaction is not particularly limited, though a small excess over the stoichiometrical amount is preferred. The amount may be decided according to the yield and economy.

The organic solvent used in this invention is, for example, a sulphur-containing polar solvent e.g. dimethyl sulfoxide, dimethyl sulfone, diphenyl sulfoxide, diphenyl sulfone, tetrahydrothiophene-1,1-dioxide (sulfolane) and a nitrogen-containing polar solvent e.g. N,N-dimethylformamide, N,N-dimethylacetamide, N-methylpyrrolidone, 1,3-dimethyl-2-imidazolidinone (DMI) and hexamethylphosphoric acid triamide (HMPA) and other non-proton polar solvents.

The reaction temperature is, in general, between 100° and 350°C. The progress of the reaction is traced by gas chromatography or thin layer chromatography and the reaction is completed when most of the starting materials have disappeared. The process of this invention is carried out by heating the 4-halogenobenzophenone of Formula I or 4,4'-dihalogenobenzophenone of the formula Ia and alkali fluoride in the organic solvent under stirring. In this case, an interlayer moving catalyst such as a large ring polyether (crown ether), quaternary ammonium salts and quaternary phosphonium salts may be added to stabilize an alkali metal cation.

According to a preferred embodiment of this invention, a small amount of benzene is added to the organic solvent containing the 4-halogenobenzophenone of Formula I or 4,4'-dihalogenobenzophenone of formula Ia and alkali fluoride and heated.

Water in the reaction system is removed in the form of an azeotropic mixture with benzene and thereafter, heating is continued at indicated temperatures under stirring, which is effected, preferably under nitrogen atmosphere to prevent the solvent from decomposing.

After the indicated reaction time, the resulting products are cooled and the final compound is extracted by an organic solvent.

Examples of the solvent for extraction include alcohols such as methanol, ethanol and isopropanol, halogenated hydrocarbons such as 1,2-dichloroethane, chloroform and carbon tetrachloride and benzenes such as benzene and toluene.

Alternatively, after completion of the reaction the solvent is distilled off under reduced pressure and then, the residues are subjected to extraction with the above extraction solvent to isolate the end product. Also, the end product may be isolated directly during the distillation under reduced pressure. If necessary, a product of high purity can be obtained by a conventional solvent recrystallization.

This invention will be illustrated by the following non-limitative examples.

Example 1

To a mixture of 10.1 g (0.04 mols) of 4,4'-dichlorobenzophenone, 9,3 g (0.16 mols) of potassium fluoride and 20 g of dimethyl sulfone were added 5 ml of benzene. Water in the reaction system was removed together with benzene by azeotropic distillation. The reaction was carried out under a nitrogen atmosphere at temperatures of 230—240°C while stirring for 45 hours.

After cooling, the reaction product was extracted twice with 15 ml of isopropanol under heating. When the joined extracts were cooled, 7.0 g of 4,4'-difluorobenzophenone was obtained.

Yield 80%, Melting point 106—107°C

Pure product was obtained by recrystallisation with a methanol/water (80/20) mixture. Melting point 106—107°C.

| Elementary Analysis | (C)% | H(%) | F(%) |
|---|---|---|---|
| Calculated | 71.5 | 3.70 | 17.4 |
| Found | 71.2 | 3.50 | 17.2 |

Example 2

To a mixture of 10.1 g (0.04 mols) of 4,4'-dichlorobenzophenone, 9.3 g (0.16 mols) of potassium fluoride and 20 g of dimethyl sulfone were added 5 ml of benzene and the water in the reaction system was removed together with benzene by azeotropic distillation. 0.5 g of Dibenzo-18-crown-6-ether were added thereto and the reaction was carried out under a nitrogen atmosphere at temperatures of 230—240°C while stirring for 30 hours. The post-treatment was effected in the same manner as in Example 1. 7.0 g of 4,4'-difluorobenzophenone were obtained.

Yield 80%, M.P. 107—107°C

Example 3

Using 13.6 g (0.04 mols) of 4,4'-dibromobenzophenone, 9.3 g (0.16 mols) of potassium fluoride and 20 g of dimethyl sulfone, 6.8 g of 4,4'-difluorobenzophenone were obtained in the same procedure as in Example 1.

Yield 78%, M.P. 106—107°C

Example 4

. To a mixture of 10.1 g (0.04 mols) of 4,4'-dichlorobenzophenone, 7.0 g (0.12 mols) of potassium fluoride and 25 g of sulfolane 5 ml of benzene were added and the water in the reaction system was removed together with benzene by azeotropic distillation.

The reaction was carried out under a nitrogen atmosphere at temperatures of 273—277°C while stirring for ten hours. After cooling the sulfolane was distilled off under reduced pressure. The residues were extracted twice with 20 ml of isopropanol under heating. When the joined extracts were cooled, 5.7 g of 4,4'-difluorobenzophenone were obtained.

Yield 65%, M.P. 106—107°C

Example 5

To a mixture of 10.1 g (0.04 mols) of 4,4'-dichlorobenzophenone, 15.2 g (0.1 mol) of cesium fluoride and 25 g of sulfolane 5 ml of benzene were added and the water in the reaction system was removed together with benzene by azeotropic distillation.

The reaction was carried out under a nitrogen atmosphere at temperatures of 200—210°C while stirring for ten hours. After cooling the post-treatment was effected in the same manner as in Example 4 and thus, 7.4 g of 4,4'-difluorobenzophenone were obtained.

Yield 85%, M.P. 106—107°C

Example 6

To a mixture of 20.2 g (0.08 mols) of 4,4'-dichlorobenzophenone, 30.4 g (0.2 mols) of cesium fluoride

and 50 g of N-methylpyrrolidone 10 ml of benzene were added and the water in the reaction system was removed together with benzene by azeotropic distillation.

The reaction was carried out under nitrogen atmosphere at temperatures of 200—202°C while stirring for 20 hours. After cooling the N-methylpyrrolidone was distilled off under reduced pressure. 12 g of 4,4'-Difluorobenzophenone were obtained by distillation under reduced pressure.

Yield 70%, M.P. 105—106°C

Boiling Point 150—155°C/12 mmHg

Pure product was obtained by recrystallization with isopropanol.

M.P. 106—107°C

Example 7

To a mixture of 10.1 g (0.04 mols) of 2,4'-dichlorobenzophenone, 4.6 g (0.08 mols) of potassium fluoride and 20 g of dimethyl sulfone 5 ml of benzene were added and the water in the reaction system was removed together with benzene by azeotropic distillation.

The reaction was carried out under a nitrogen atmosphere at temperatures of 230—240°C while stirring for 11 hours. After cooling the reaction product was extracted under heating twice with 20 ml of isopropanol. The extracts together were cooled and thus, 7.5 g of 2-chloro-4'-fluorobenzophenone were obtained.

Yield 80%, M.P. 60—61°C

Pure product was obtained by recrystallization with isopropanol.

M.P. 60—61°C

Example 8—13

Using 0.04 mols of 4-chlorobenzophenone derivatives, the reaction was carried out in the same procedure as in Example 7 to obtain the corresponding 4-fluorobenzophenone derivatives. The results obtained are set forth in Table 1.

TABLE 1

| Ex. No. | 4-Fluorobenzophenone derivatives | Yield (%) | M.P. (°C) |
|---|---|---|---|
| 8 | 4-Fluorobenzophenone | 86 | 49—50 |
| 9 | 4-Fluoro-3'-methylbenzophenone | 81 | 97—98 |
| 10 | 4-Fluoro-4'-n-butylbenzophenone | 78 | 75—76 |
| 11 | 4-Fluoro-4'-phenylbenzophenone | 80 | 148—149 |
| 12 | 4-Fluoro-4'-ethoxybenzophenone | 72 | 87—88 |
| 13 | 4-Fluoro-3'-trifluoromethyl-benzophenone | 82 | 100—101 |

Example 14

To a mixture of 12.2 g (0.05 mols) of 4-chloro-2',4'-dimethylbenzophenone, 12.1 g (0.08 mols) of cesium fluoride and 20 g of N-methylpyrrolidone were added 5 ml of benzene and the water in the reaction system was removed together with benzene by azeotropic distillation. The reaction was carried out under nitrogen atmosphere by heating under reflux while stirring for 15 hours. After cooling, the reaction product obtained was poured into 100 ml of water and then, extracted with 50 ml of diethyl ether and further with 50 ml of ether. The extracts together were dried and distilled and thus, 8.9 g of 4-fluoro-2',4'-dimethylbenzophenone were obtained.

Yield: 78%, B.P. 151—152°C/10 mmHg

Example 15

To a mixture of 14.4 g (0.05 mols) of 4-bromo-2',5'-dimethylbenzophenone, 5.5 g (0.095 mols) of potassium fluoride, 0.06 g (0.005 mols) of cesium fluoride and 20 g of sulfolane were added 5 ml of benzene and the water in the reaction system was removed together with benzene by azeotropic distillation.

The reaction was carried out under a nitrogen atmosphere at temperatures of 245—250°C while stirring for 15 hours. After cooling, the reaction products were poured into 100 ml of water and then, extracted with 50 ml of diethyl ether and further with 50 ml of ether. The extracts together were dried and distilled and thus, 7.8 g of 4-fluoro-2',5'-dimethylbenzophenone were obtained.

Yield 69%, B.P. 164—166°C/10 mmHg

**Claims**

1. A process for producing 4-fluorobenzophenone derivatives which comprises effecting a halogen-fluorine exchange reaction between 4-halogenobenzophenone derivatives of Formula I,

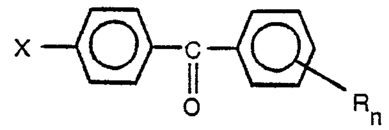

I

wherein X is chlorine, bromine or iodine atom, R is hydrogen, an alkyl group, an aralkyl group, a cycloalkyl group, an aryl group, an alkoxy group, an aryloxy group, an alkylthio group, a carboalkoxy group, an acetyl group, a trifluoromethyl group, a nitro group or a chlorine, bromine or iodine atom, n is an integer of 1—5 and when n is 2 or more, R is the same or different, and an alkali fluoride in an organic solvent at a temperature of 100°C to 350°C.

2. The process of claim 1, wherein the halogen-fluorine exchanging reaction is carried out between a 4,4′-dihalogenobenzophenone of the formula Ia

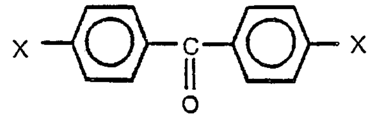

Ia

and an alkali fluoride, wherein X is chlorine, bromine or iodine.

3. The process of claim 1 or 2 wherein said alkali fluoride is at least one member selected from lithium fluoride, potassium fluoride, sodium fluoride, rubidium fluoride and cesium fluoride.

4. The process of claim 1 or 2 wherein said halogen-fluorine exchanging reaction is carried out in the presence of an interlayer moving catalyst.

**Patentansprüche**

1. Verfahren zur Herstellung von 4-Fluorbenzophenon-Derivaten, gemäß dem eine Halogen-Fluor-Austauschreaktion zwischen 4-Halogenbenzophenon-Derivaten der Formel (I)

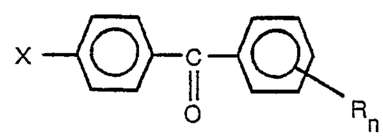

I

in der X ein Chlor-, Brom- oder Iodatom, R Wasserstoff, eine Alkylgruppe, eine Aralkylgruppe, eine Cycloalkylgruppe, eine Arylgruppe, eine Alkoxygruppe, eine Aryloxygruppe, eine Alkylthiogruppe, eine Carbalkoxygruppe, eine Acetylgruppe, eine Trifluormethylgruppe, eine Nitrogruppe oder ein Chlor-, Brom- oder Iodatom ist, n eine ganze Zahl von 1 bis 5 bedeutet und wenn n 2 oder mehr ist, R gleich oder verschieden sind, und einem Alkalifluorid in einem organischen Lösungsmittel bei einer Temperatur von 100 bis 350°C durchgeführt wird.

2. Verfahren nach Anspruch 1, bei dem die Halogen-Fluor-Austauschreaktion zwischen einem 4,4′-Dihalogenbenzophenon der Formel Ia

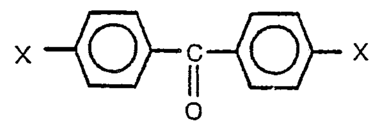

Ia

und einem Alkalifluorid durchgeführt wird, wobei X Chlor, Brom oder Iod bedeutet.

3. Verfahren nach Anspruch 1 oder 2, bei dem das Alkalifluorid mindestens eine Verbindung aus der Gruppe Lithiumfluorid, Kaliumfluorid, Natriumfluorid, Rubidiumfluorid und Caesiumfluorid ist.

4. Verfahren nach Anspruch 1 oder 2, bei dem die Halogen-Fluor-Austauschreaktion in Gegenwart eines Grenzflächenmobilen Katalysators durchgeführt wird.

**Revendications**

1. Procédé de production de dérivés de fluoro-4 benzophénone, qui consiste à effectuer une réaction déchange halogène/fluor, dans un solvant organique à une température de 100° à 350°C, entre un fluorure

alcalin et des dérivés d'halogèno-4 benzophénone de formule I,

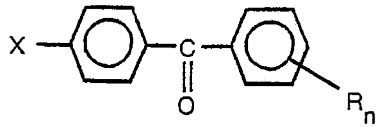

I

dans laquelle X est un atome de chlore, de brome ou d'iode, R est de l'hydrogène, un groupement alkyle, un groupement aralkyle, un groupement cycloalkyle, un groupement aryle, un groupement alkoxy, un groupement aryloxy, un groupement alkylthio, un groupement carboalkoxy, un groupement acétyle, un groupement trifuorométhyle, un groupement nitro, ou un atome de chlore, de brome ou d'iode, n est un nombre entier de 1 à 5, et lorsque n est égal ou supérieur à 2, les R sont identiques ou différents.

2. Procédé selon la revendication 1, dans lequel la réaction d'échange halogène/fluor s'effectue entre une dihalogèno-4,4' benzophénone de formule Ia,

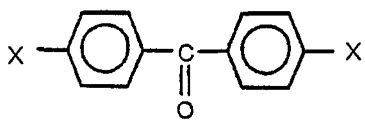

Ia

et un fluorure alcalin, X étant chlore, brome ou iode.

3. Procédé selon la revendication 1 ou 2, dans lequel le fluorure alcalin est au moins un membre choisi à partir des fluorures de lithium, de potassium, de sodium, de rubidium et de césium.

4. Procédé selon la revendication 1 ou 2, dans lequel la réaction d'échange halogène/fluor s'effectue en présence d'un catalyseur en intercouche mobile.